# EUROPÄISCHE PATENTANMELDUNG

(11) **EP 1 695 737 A1**
(43) Veröffentlichungstag der Anmeldung: **30.08.2006**
(21) Anmeldenummer: 05028504.8
(22) Anmeldetag: 27.12.2005
(51) Int. Cl.: A61N 2/06, A61H 39/00, A61K 41/00

(54) **Vorrichtung zur Übertragung energetischer Schwingungsinformationen**

(30) Priorität: 25.02.2005 AT 3252005
(71) Anmelder: Quintsysteme für holopathische Medizin Ges.m.b.H., A-3107 St. Pölten (AT)
(72) Erfinder: Dillinger, Klaus, Dipl.-Ing., 3100 St. Pölten (AT); Steiner, Christian, Dr., 9073 Viktring (AT)
(74) Vertreter: Grabherr, Claudia

(57) **Zusammenfassung**

Die Erfindung betrifft eine Vorrichtung zur Übertragung energetischer Schwingungsinformationen bestehend aus einem Permanentmagneten (1), wobei mit dem Permanentmagneten (1) ein energetisch informiertes Medium (2) verbunden ist.

## Beschreibung

Die Erfindung betrifft eine Vorrichtung zur Übertragung energetischer Schwingungsinformationen mittels eines Permanentmagneten, der mit einem energetisch informierten Medium verbunden ist, insbesondere zur energetischen Stimulation von Organismen und zur Energetisierung von Speisen, Getränken und Kosmetika.

Es ist bekannt, dass das Energiesystem von Organismen, das unter anderem als Meridiansystem oder Chakrensystem beschrieben wird, durch energetische Schwingungsinformationen beeinflusst werden kann. Auf diesem Wirkprinzip beruhten beispielsweise die klassische Homöopathie aber auch eine Vielzahl von moderneren energetischen Therapieverfahren wie Elektroakupunktur, Bioresonanz oder Bachblütentherapie. Bei diesen Verfahren wirken die Schwingungsspektren bestimmter Substanzen, wie beispielsweise Heilkräutern, Mineralstoffen, Nahrungsergänzungen oder homöopathisierten Schadstoffen auf das Energiesystem ein und bewirken dessen Harmonisierung und Regenerierung.

Die Substanzschwingungen können bekanntermaßen auf verschiedene Arten appliziert werden. Bekannt sind beispielsweise das Anwendungsprinzip der Homöopathie, nämlich die direkte Applikation alkoholischer Verdünnungen der Ausgangssubstanz oder die elektromagnetische Übertragung der Substanzinformationen wie bei Bioresonanz und Elektroakupunktur. Das genaue Wirkprinzip dieses Mechanismus ist wissenschaftlich nicht einwandfrei geklärt, doch gilt als gesichert, dass lebende Organismen auf derartige niederenergetische Informationen reagieren. Allgemein wird angenommen, dass die charakteristischen energetischen Substanzinformationen in das thermische Rauschen eingekoppelt sind, welches jeder Körper bei Temperaturen über dem absoluten Nullpunkt abgibt.

Weiters sind Vorrichtungen bekannt, mit denen energetische Schwingunginformationen von Substanzen auf bestimmte energetische Trägermedien kopiert werden können. Als geeignete Zielmedien sind beispielsweise diverse Flüssigkeiten, Folien aus Metallpigment oder Kunststoff oder bestimmte Metallegierungen bekannt. Die übertragenen Informationen bleiben in diesen Materialen dauerhaft gespeichert, wobei die Haltbarkeit der Information einerseits vom Material des Mediums und andererseits von der Umgebung abhängt.

Da diese Medien die gespeicherten Substanzinformationen nur über ihr termisches Rauschen abstrahlen, ist die von ihnen abgegebene energetische Information nur sehr schwach. Für viele praktische Anwendungen werden diese Informationen daher auf elektronischem Wege verstärkt. Auf dieser Basis können elektronische energetische Therapiegeräte realisiert werden, die der Anwender zur Erzielung einer medizinischen Wirkung meist lange Zeit direkt am Körper tragen muss. Diese Lösung hat aber den Nachteil, dass man dafür ein elektronisches Gerät mit einer Energiequelle benötigt, welches in der Praxis wegen seiner Größe und seines Gewichts nicht komfortabel zu handhaben ist. Außerdem sind diese Geräte auch meist sehr teuer.

Aufgabe der vorliegenden Erfindung ist es, einen effiziente, kostengünstige und komfortabel handhabbare Lösung ohne Verwendung eines elektronischen Geräts für die energetische Schwingungsübertragung zu schaffen.

Diese Aufgabe wird erfindungsgemäß gelöst durch Verbindung eines Permanentmagneten mit einem geeigneten energetischen Medium. Durch das vom Permanentmagneten erzeugte Magnetfeld werden die im energetischen Medium gespeicherten Schwingungsinformationen auf den Anwender übertragen. Mit der Erfindung soll also keine herkömmliche Magnetfeldtherapie durchgeführt werden, sondern eine Vorrichtung geschaffen werden, welche das Feld eines Permanentmagneten als Transportmedium und Verstärker für energetische Informationen nützt. Dies erfolgt erfindungsgemäß dadurch, dass ein energetisch entsprechend vorbereiteter Informationsträger mit einem plättchenförmigen Permanentmagneten verbunden wird.

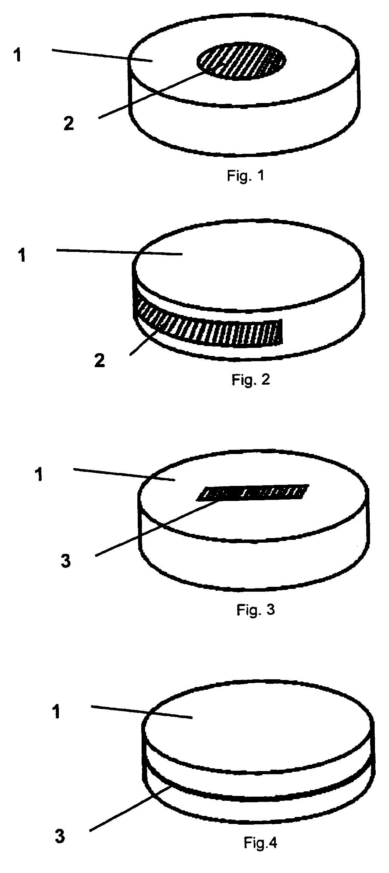

In der Zeichnung ist der Erfinungsgegenstand beispielsweise dargestellt. Es zeigen die Fig. 1 bis 4 verschiedene Ausführungen der Erfindung.

Der Permanentmagnet 1 kann beispielsweise ein Stabmagnet mit einem Durchmesser von 10 mm und einer Höhe von 5 mm sein. Die magnetische Flussdichte Feldstärke des Magneten ist für das Prinzip der Erfindung ohne Belang. Die besten Resultate wurden mit Flussdichten zwischen 140 und 300 mT erzielt.

Das mit dem Permanentmagneten verbundene energetische Medium kann in Form und Material verschieden ausgeprägt sein. In einer Ausführungsform der Erfindung besteht das energetische Medium aus einer dünnen Metall-, Metallpigment- oder Kunststoff-Folie 2. Besonders gute Resultate wurden mit einer Folie aus dem Kunststoff Polyimid erzielt. Die Folie 2 wird entweder auf einer oder beiden Planarflächen oder auf der Mantelfläche des Stabmagneten 1 aufgeklebt. In einer anderen Ausführungsform der Erfindung besteht das energetische Medium aus einem Metalldraht 3, vorzugsweise aus Gold, Platin oder Silber. Der Draht wird entweder auf einer oder beiden Planarflächen oder auf der Mantelfläche des Stabmagneten 1 durch Löten, Kleben oder Einfräsen dauerhaft befestigt.

Auf diese Weise können Medien mit beliebiger energetischer Prägung mit dem Permanentmagneten verbunden werden. Das Feld des Permanentmagneten übernimmt die energetischen Informationen des Mediums und strahlt sie auf den Anwender ab. Dadurch wird das Meridiansystem des Anwenders im Sinne der energetischen Informationen des Mediums beeinflusst, es erfolgt also eine energetische Therapie. Durch die Kombination mit einem Magnetfeld ergibt sich eine um Größenordnungen stärkere Wirkung verglichen mit der Anwendung eines energetischen Mediums allein ohne Kombination mit Magnetfeld.

Die therapeutische Wirksamkeit kann durch Wahl eines geeigneten Volumens des energetischen Mediums (d.h. Fläche und Dicke der Folie 2 bzw. Länge und Durchmesser des Drahtes 3) im Verhältnis zum Trägermagneten 1 bzw. durch Variation der Feldstärke des Magneten 1 variiert werden.

Das energetische Medium wird vor Aufbrinung auf den Trägermagneten mittels eines der bekannten Verfahren und Vorrichtungen geeigneten energetischen Informationen bespeichert. Solche Vorrichtungen sind beispielsweise beschrieben im europäischen Patent EP0495132 oder im österreichischen Gebrauchsmuster AT000130U2. Mit derartigen Vorrichtungen können im Prinzip beliebige energetische Informationen auf energetische Medien übertragen werden. Diese Informationen werden im energetischen Medium dauerhaft gespeichert. Die Haltbarkeit der Informationsspeicherung hängt vom Material des Mediums ab. Für Polyimid-Folien beträgt die Haltbarkeit erfahrungsgemäß mehr als ein Jahr, für Drähte aus einer Goldlegierung mindestens 15 Jahre.

Die Erfindung kann sowohl bei Menschen als auch bei Tieren angewendet werden. Für die Anwendung wird die erfindungsgemäße Vorrichtung direkt am Körper befestigt. Die optimale Körperstelle für die Anwendung kann je nach Anwendungszweck varüeren. Im allgemeinen wird die Vorrichtung über bestimmten Akupunkturpunkten oder Reflexzonen des Körpers befestigt. Die Befestigung kann durch Aufkleben mithilfe eines Pflasters oder mit anderen Befestigungsvorrichtungen erfolgen. Es ist dabei nicht notwendig, dass die erfindungsgemäße Vorrichtung in direktem Hautkontakt steht. Sie kann daher auch in Befestigungsvorrichtungen oder Gehäuse aus beliebigen Materialien (z. B. Kunststoff, Metall, Leder) eingebaut oder in Schmuck- bzw. Kleidungsstücke integriert werden.

In einer weiteren Ausführungsform kann die Erfindung auch zur Übertragung energetischer Schwingungsinformationen auf Lebensmittel, Getränke, Flüssigkeiten, Kosmetika u. dgl. eingesetzt werden. Dazu wird einfach der Behälter mit dem Empfängermedium auf die erfindungsgemäße Vorrichtung gestellt. Nach einer gewissen Einwirkzeit übertragen sich die energetischen Informationen vom erfindungsgemäßen Gerät auf das Zielmedium, wobei die Einwirkzeit von der Flussdichte des verwendeten Permanentmagneten, vom Abstand zum Behälter mit dem Zielmedium und von der geometrischen Anordung abhängen. Typische Einwirkzeiten liegen zwischen 10 Sekunden und 15 Minuten. Auf diese Weise ist es möglich, beliebig energetisierte Speisen und Getränke bzw. beliebig energetisierte Kosmetika herzustellen. Diese haben dann - je nach der übertragenen Schwingungsinformation - einen stimulierenden, ausgleichenden oder heilenden Einfluss auf das Energiesystem des Anwenders.

Durch eine geeignete Auswahl der auf das Medium zu speichernden energetischen Informationen kann beim Anwender eine große Bandbreite von energetischen und therapeutischen Effekten erzielt werden. Mögliche Anwendungen in der Humanmedizin sind beispielsweise eine allgemeine Vitalitätssteigerung und Verbesserung des Wohlbefindens, die Behandlung von Schmerzen im Bewegungsapparat und die Beschleunigung der Wundheilung bei Verletzungen oder nach Operationen. Eine mögliche Anwendung in der Tierzucht ist die energetische Stimulation von Masttieren mit dem Ziel einer positiven Beeinflussung von Fleischmenge und -qualität.
Fig. 1 zeigt eine Ausführungsform der Erfindung, bei welcher das energetische Medium in Form einer Folie 2 ausgeführt ist, die auf einer Planarfläche des Permanentmagneten 1 angeordnet ist.
Fig. 2 zeigt eine Ausführungsform der Erfindung, bei welcher das energetische Medium in Form einer Folie 2 ausgeführt ist, die auf der Mantelfläche des Permanentmagneten 1 angeordnet ist.
Fig. 3 zeigt eine Ausführungsform der Erfindung, bei welcher das energetische Medium in Form eines Metalldrahtes 3 ausgeführt ist, der auf einer Planarfläche des Permanentmagneten 1 angeordnet ist
Fig. 4 zeigt eine Ausführungsform der Erfindung, bei welcher das energetische Medium in Form eines Metalldrahtes 3 ausgeführt ist, der auf der Mantelfläche des Permanentmagneten 1 angeordnet ist

## Patentansprüche

1. Vorrichtung zur Übertragung energetischer Schwingungsinformationen bestehend aus einem Permanentmagneten (1), **dadurch gekennzeichnet, dass** mit dem Permanentmagneten (1) ein energetisch informiertes Medium (2, 3) verbunden ist.

2. Vorrichtung nach Anspruch 1, **dadurch gekennzeichnet, dass** das energetisch informierte Medium in Form einer Folie (2) aus Kunststoff oder Metall oder eines Metalldrahtes (3) ausgebildet ist.

3. Vorrichtung nach den Ansprüchen 1 und 2, **dadurch gekennzeichnet, dass** die Folie (2) aus dem Kunststoff Polyimid besteht.

4. Vorrichtung nach den Ansprüchen 1 und 2, **dadurch gekennzeichnet, dass** der Draht (3) aus einer Metalllegierung mit einem Goldanteil von über 50% besteht.
